# EUROPEAN PATENT SPECIFICATION

(11) **EP 3 158 051 B1**
(45) Date of publication and mention of the grant of the patent: **07.04.2021**
(21) Application number: 15727041.4
(22) Date of filing: 21.05.2015
(51) Int. Cl.: C12M 1/02

(54) **MICROBIAL FERMENTATION SYSTEM FOR GROWING AND DISCHARGING A BIOLOGICAL MATERIAL**
MIKROBIELLES FERMENTIERUNGSSYSTEM ZUR ZÜCHTUNG UND ENTLADUNG EINES BIOLOGISCHEN MATERIALS
SYSTÈME DE FERMENTATION MICROBIENNE POUR CULTIVER ET ÉVACUER UN MATÉRIAU BIOLOGIQUE

(30) Priority: 17.06.2014 GB 201410806
(43) Date of publication of application: 26.04.2017
(73) Proprietor: NCH Corporation, Irving, Texas 75062 (US)
(72) Inventor: CULPIN, David, Bramcote, Nottinghamshire NG9 3DG (GB); MOORE, Lawrence, Chichester, Sussex PO20 3UA (GB)
(74) Representative: Wolstenholme, Daniel
(86) International application number: PCT/GB2015/051506
(87) International publication number: WO 2015/193635

(56) References cited:
- WO-A2-2005/017178
- US-A- 3 984 286
- US-A1- 2005 054 086

## Description

This invention relates to a system for microbial fermentation. This system overcomes the negative aspects of the lag phase in biological growth of a starter material by promoting the exponential growth phase.

There are devices and systems available which grow, feed, solubilise, transfer and dispense bacteria for various applications. These microbial fermentation systems are available in a range of sizes, depending on the desired application. Applications can include, for example, digestion and removal of grease from food processing or restaurant drains, grease traps, grease interceptors, abattoirs, sewers, septic tanks and sewage treatment plants. The microbial fermentation systems are often designed to deliver large amounts of active, naturally occurring bacteria to the desired application (e.g. a drain). The bacteria help to accelerate the natural decomposition of organic waste US3984286 discloses an apparatus and a process of using the apparatus for microbial growth comprising: a pre-fermenter; an intermediate fermenter connected in series, by a first conduit, with and downstream of said pre-fermenter, whereby effluent from said pre-fermenter is fed to said intermediate fermenter, said intermediate fermenter having a volume substantially larger than said pre-fermenter. A first heat exchange means is in heat transfer relation with said intermediate fermenter.

Stringent purity requirements are usually set for wastewater emerging from industrial and commercial facilities and microbial fermentation systems such as these are capable of producing sufficient bacteria to transform highly polluted wastewater into wastewater that meets regulatory requirements. These microbial systems are also low in energy consumption and have the additional benefit of reducing the amount of off-site sludge disposal. The use of bacteria to process the waste can replace the use of harmful chemicals.

Bacterial growth occurs in the following stages (also known as the bacterial growth curve):
1. Lag phase; a delay before activity and growth is observed
2. Growth; exponential growth phase
3. Plateau; where number are fairly constant
4. Decline; where numbers decrease at an increasing rate (also known as the death phase)

Current devices grow bacteria which are dormant or in a low activity state, therefore every recycle includes a lag phase. The exponential growth phase is affected by the process conditions. This means that a significant part of the growth cycle is inefficient.

According to the present invention there is provided a system for producing an aqueous bacteria-containing slurry, the system comprising a water supply, a control system, and at least two tanks, where at least a first tank is a primary phase tank, and a second is a secondary phase tank, the primary phase tank being in fluid communication with the secondary phase tank, where the secondary phase tank further comprises a product outlet characterised in that the system further comprises a recirculation loop to the secondary phase tank.

The primary phase is the first stage from the bacterial growth curve, which is the lag phase. The secondary phase is the exponential growth phase of the bacterial growth phase.

The system according to the present invention prepares and maintains the ideal conditions for the exponential growth phase. The bacteria or consortium of bacteria that have completed the lag phase and are beginning the growth phase are transferred from the primary phase tank(s) to the secondary phase tank. Preferably, the transfer is conducted in a way and at a time that minimises transfer shock, which could otherwise retard growth. The system ensures the correct process conditions are used to extend the time and expand the volume of the exponential growth phase of the input bacteria to provide large scale generation in the secondary phase tank, which can act as a secondary incubation process. The secondary phase can also be further extended into tertiary and further stages as required. Additional tanks for either the primary phase or secondary phase may be arranged in series or in parallel. The product is a liquid or slurry containing bacteria in the active state, which can be immediately used for the desired application.

The system can be used with aerobic bacteria, anaerobic bacteria or a combination of aerobic and anaerobic bacteria. If aerobic bacteria are used, the system can further include a device or system for aerating the fluid in which the bacteria is grown. If anaerobic bacteria are used, no aeration step is required.

The bacteria can be pre-incubated in any number of existing devices, tailored to suit the particular bacterial strains required for the end application. The conditions in the system can be adjusted to match those required by the input bacteria/bacterial strain(s) and to suit the process task.

There are a number of conditions that need to be controlled during the fermentation process. It is desirable to control the volume (in order to avoid competitive stress), the aeration levels (degree of oxygenation), the temperature, the pH, the nutrition levels and homogeneity of the mixture.

The temperature of the process is one of the more influential physical factors. The temperature range can be between -10°C to 95°C, although more typically, the temperature range is between 10°C and 50°C. The optimum temperature range is determined by the specific species of bacteria used. Psychrophiles exhibit optimum growth at temperatures below 20°C, mesophiles at temperatures in the range of 20-45°C, and thermophiles at temperatures of above 45°C. Extremophiles can survive at temperatures of -17°C and 130°C. Most of the commonly used bacterial cultures are mesophiles, and the temperatures of the tanks are controlled between the 10°C and 50°C range.

The pH of the process is another important factor. Most bacteria prefer a pH in the range of 5-9, with an optimum pH near 7. Anaerobic bacterial can tolerate a wider pH range than aerobic bacteria.

Although many types of bacteria can only grow in the absence of oxygen (anaerobic), many types are capable of growing in either the presence or absence of oxygen. Aerobic organisms rely on the presence of oxygen for enzymatic reactions and for the electron transport system that they use to generate energy.

The growth medium can be formulated specifically for the species of bacteria that is to be used. This can be achieved by blending pure compounds in precisely defined proportions to yield a well-defined synthetic growth medium. Synthetic growth mediums tend to be easily reproduced, have low foaming tendency, show translucency and allow easy recovery of the product. The synthetic growth medium may be aqueous, and can comprise of water with a selected group of nutrient additives. Alternatively, a naturally occurring growth media (complex media) can be used. These types of growth media include molasses and corn steep liquor and the chemical composition of naturally occurring growth media may not be precisely defined due to their complex nature.

In addition to an energy source and a carbon source, the bacteria may also require additional nutrients, including essential and trace elements. The nutrients required depend on the species of bacteria, but include sources of nitrogen and sulphur, as well as minerals and vitamins. If additional nutrients are added to the system, the aim is usually to compensate for low quantities of a certain element or ingredient in the growth media.

It is possible to use a single strain of bacteria or alternatively a consortium of bacteria can be used. The bacteria are provided in the form of a 'starter material'. This starter material can be a particulate solid comprising a bacterial component. It can be provided in the form of pellets, prills, tablets or granules, as well as in liquid form. The starter material may also contain a nutrient component. When the starter material is provided in solid form, it may be preferable to add the material to the one or more tanks using a gravity feed mechanism.

The feed and product streams can be continuous or periodic depending on the best growth requirements for the bacteria being used. Other factors which would influence whether a batch, periodic or continuous process is used are: the type of plant, the process conditions and the processing regime.

More than two primary phase tanks can be used to prepare the bacteria. The bacteria-containing starter material is added to the primary phase tank(s), where it is held until the lag phase is complete or almost complete. The primary phase tank(s) can be heated or cooled as required. The primary phase tank(s) may also contain mixing apparatus to ensure a homogeneous mixture and good aeration levels. Once the lag phase is complete or near complete, the prepared bacteria, which are approaching the growth phase, can be transferred into the secondary phase tank. Nutrients can be added to any of the primary phase or secondary phase tanks if required.

The primary phase tank(s) can be any suitable vessel for preparing the bacteria and holding the aqueous mixture or sludge until the lag phase is almost complete. Although described as a tank throughout this application, the vessel could also be a drum, bowl, bucket, bottle, a sump or any other vessel that can be used for the purposes of holding fluid. Preferably, the primary phase tank has an outlet which feeds into the secondary phase tank.

The secondary phase tank can also be any suitable vessel for the purposes of holding fluid. Preferably, the secondary phase tank has at least one inlet for receiving the bacteria-containing fluid from the primary phase tank and at least one outlet for supplying the product to the desired application. The secondary tank may, for example, be a mixing tank of the type disclosed in WO2011/005605, the features of which are incorporated herein in their entirety.

The primary phase tanks and the secondary phase tanks may be any size which is suitable for the intended application. For example, the primary and secondary phase tanks may have a fluid capacity in the range of 250ml to 50,000 litres.

The tanks are fitted with temperature sensors and means for heating or cooling the contents of the tank to the desired temperature. The temperature of the contents of the tank can be altered by using a warm water feed, a heating jacket, a heat exchanger within the tank, or alternatively a heat exchanger positioned on one of the recirculation or feed lines. It is also possible to use one or more thermoelectric heating device(s) suspended within the tank. The temperature sensors, and the means for heating/cooling the tanks can all be linked to a control system. The tanks can be arranged such that the temperature of the primary phase tanks is controlled independently of the temperature of the secondary phase tanks. Alternatively, the temperature of each tank could be individually controlled.

The control system could comprise a programmable logic controller (PLC) or a microprocessor. For example, the control system may comprise a microprocessor that is connected to the solenoid valves in order to control the flow of water to the secondary phase tank. The control system can be programmed to operate at designated times for predetermined and preset intervals. Alternatively, the control system can be designed to respond to readings from sensors, to maintain preset conditions. The control system can also be used to activate the feed of the 'starter material' into the primary phase tank, the flow of water into the primary and secondary phase tanks, and to control the flow of the prepared bacterial culture from the primary into the secondary phase tank. In addition, the control system could control the flowrate and volume of any recirculating streams, any mixing devices and any aeration devices. In the example described below, the rate of recirculation affects the aeration of the fluid in the secondary phase tank, and therefore by controlling the flow rate of the recirculating stream, the aeration of the liquid is controlled. Any available source of power can be used.

The valves used on the system would typically be control valves; capable of being controlled by the control system and linking into a feedback loop. Globe or diaphragm valves are most suitable for use as control valves, and the valves may be solenoid valves (which use a diaphragm to provide smooth control over the full range of flow). The valves used for the water inlet stream are typically non-return valves in order to avoid contamination of the clean water supply. It may not be necessary to have a non-return valve or air break on the inlet stream, depending on the quality of the water supply and local regulations. Alternative types of valves include gate valves, butterfly valves, ball and plug valves. The number of valves used, the type of valve and the placement of these valves can vary with each system. The valve location can depend, for example, on whether more than one primary or secondary phase tank is used, whether nutrients are added from a separate nutrient tank, and on whether an aeration method is used, and if so, which type. The types of pump used can also vary, depending on the solid content of the recycle loop and product stream, for example. Submersible pumps can also be used, if necessary. Alternatively, gravity feed systems may be used in place of pumps.

The liquid level within the tank can be controlled by the control system. The control system could include level sensors or detectors within the tank. An example of a suitable level sensor would be a float switch. Alternatively, flow meters can be used to calculate the level within the primary or secondary phase tank.

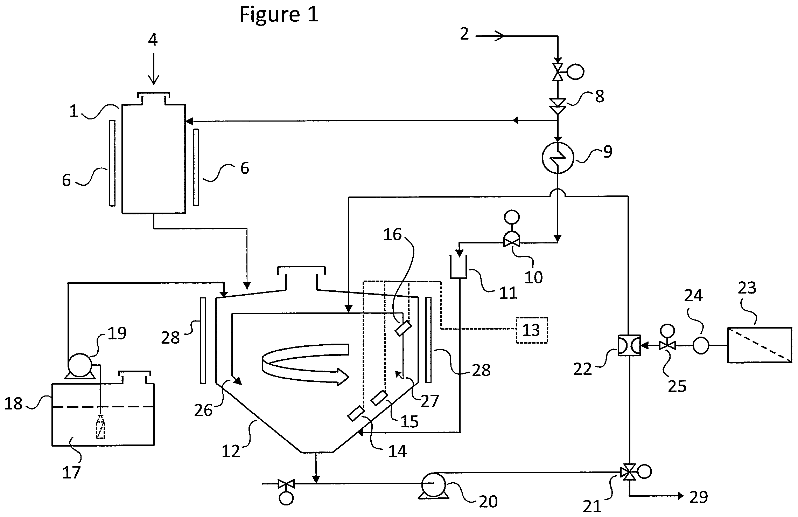

An example of a system utilizing aerobic bacteria is described below, with reference to Figures 1-3. This example includes the use of an eductor located in a recirculating fluid stream to the secondary phase tank. The eductor can be used to improve the aeration of the liquid stream. It is possible to replace the eductor with any other device capable of entraining bubbles of air into the liquid stream, at other locations in the process. The eductor is effectively an ejector or injector unit, which relies on the Venturi effect to use the energy of a moving fluid to create a low pressure zone which draws in and entrains a suction fluid (in this example, air). The entrainment of air results in an aerated moving fluid stream. Other alternative methods for entraining the liquid include; aeration turbines, fine bubble diffusers, coarse bubble diffusers and linear aeration tubing, or passing the liquid through air by means of fountains, cascades, paddle-wheels or cones. Smaller bubbles result in increased gas transfer efficiency, as more gas is exposed to the liquid (increased contact surface). Diffusers or spargers can also be designed into the system to cause turbulence or to promote mixing.

Examples of the present invention will now be described, by way of example only, with reference to the accompanying diagrammatic drawings, in which:
Figure 1 is a flow diagram of one example of a system for growing and discharging a biological material; where one primary phase tank and one secondary phase tank is used.
Figure 2 is a perspective view of the secondary phase tank, illustrating the use of a tangential flow nozzle arrangement to create a swirling vortex.
Figure 3 is a plan view of the secondary phase tank, illustrating the circulation induced in the secondary phase tank by the tangential positioning of the nozzles.

Figure 1 illustrates an example of a system for preparing an aqueous bacteria-containing slurry, where a single primary phase tank 1 is used with a single secondary phase tank 2.

A water supply 2 is provided to primary phase tank 1, via a non-return valve 3. A source of bacteria-containing starter material 4 is added to the primary phase tank 1 through inlet 5. In this example, the starter material is a solid, in pellet form. Additional nutrients and a growth medium may also be added. The water supply may be heated before being supplied to the primary phase tank 1, although this is not shown in this example. In this example, primary phase tank 1 is heated using a heating jacket 6. The temperature and pH of the contents of the primary phase tank 1 are carefully controlled to encourage the growth of the bacteria. In this way, primary phase tank 1 acts as an initial incubation stage for the bacteria.

When the bacteria in primary phase tank 1 are approaching the end of the lag phase and are starting to exhibit growth, the bacteria-containing liquid can be transferred to the secondary phase tank 2 via outlet 7. The output stream from primary phase tank 1 is fed into the secondary phase tank 2 where it is mixed with preheated water supplied at a temperature of 20°C to 30°C. The inlet water 2 passes through a non-return valve 8 and is heated in a heat exchanger or by a heater 9. The warm water then passes through a solenoid valve 10 and an air gap/break 11 before entering the secondary phase tank 12. The purpose of the air break 11 is to prevent back-flow and to comply with water regulations (as typically imposed by local authorities).

The level of liquid in the secondary phase tank 12 can be controlled using a plurality of valves, which may be solenoid valves, in conjunction with a programmable control system 13 and multiple level detectors within the secondary phase tank 12. The control system may be designed to able to respond to fluctuations in the liquid level within the tank by using level detectors, for example, float switches 14, 15, 16.

Nutrients 17 are fed to the secondary phase tank 12 from a nutrient tank 18, using a pump 19. Alternatively, the nutrients may also be fed into the secondary phase tank 2 using a gravity feed system. The nutrient feed may be a solid material, a liquid, or a sludge. The pump 19 or the inlet valve (not shown) can be linked to the control system in order to allow control over the quantity of nutrient added to the secondary phase tank 12.

The liquid from the secondary phase tank 12 in this example is recirculated from the tank in order to improve the homogeneity of the mixture, and to improve aeration levels. When the secondary phase tank 12 is partially full, the pump 20 is started. The pump 20 can draw liquid from the bottom of the secondary phase tank 12 via a three-way control valve 21 to control the level of fluid in the secondary phase tank. As an alternative to a pump, a gravity feed system may be used. The liquid is drawn from the secondary phase tank 12 and passed through an eductor 22, which sucks air through a filter 23. The flow of air can be controlled using a flow meter 24 fitted with a control valve 25. Controlling the amount of air entering the liquid stream allows the optimisation of the dissolved oxygen levels in the secondary phase tank 12 (and therefore allows the control of the aeration, one of the process parameters). The oxygen-enriched liquid is injected into the circulating liquid inside the secondary phase tank 12 through nozzles 26 and 27. The two nozzles 26 and 27 are arranged tangentially within the secondary phase tank. The aerated liquid is recirculated until the top of the exponential growth curve for the particular bacteria or bacterial consortium is reached. The nature and quality of aeration and mixing can be improved by the use of this arrangement.

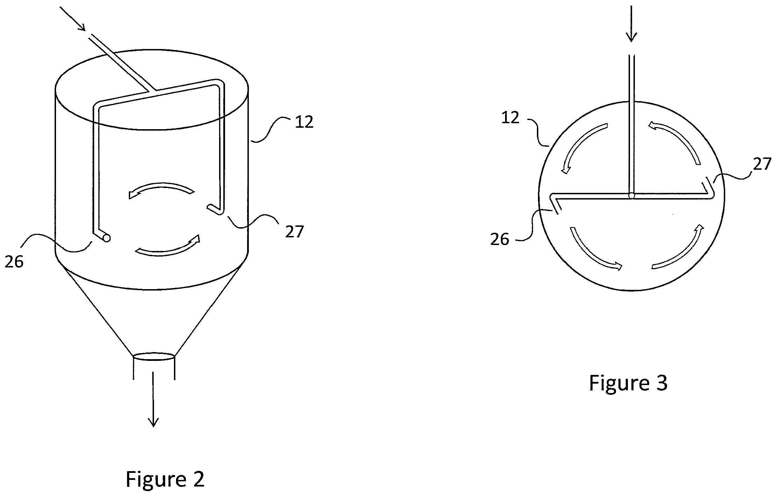

The effect of the tangential nozzles 26 and 27 within the secondary phase tank 12 is illustrated in Figure 2 and Figure 3. The manner of liquid injection through tangential nozzles creates a swirling vortex inside the secondary phase tank 12, which keeps the microscopic air bubbles suspended in the liquid and prolongs the contact time with the liquid. This helps to maximise the transfer of oxygen into the liquid and maintains the dissolved oxygen level at the ideal level for maximum growth of the bacterial culture.

The temperature in the tank is maintained at the temperature most suitable for the growth of the bacteria. A heating jacket 28 can be used to maintain this optimum temperature. Alternatively any suitable heat exchanger arrangement could be used, for example, a thermoelectric heating/cooling system.

When exponential growth has been achieved, the three-way control valve 21 can be used to discharge the product 29, a highly active bacterial culture, to the process or desired application. The pump 20 can be used to assist with recovery of the product 29 from the secondary phase tank.

The secondary phase tank 12 can be arranged in order to allow the continuous feed of bacteria from the primary phase tank 1 into the secondary phase tank 12. An equilibrium in growth rate can be achieved in the secondary phase tank 12 by controlling the rate of the feed of the bacteria into the secondary phase tank 12. A suitable level of nutrient 17 is maintained in the secondary phase tank 12. A corresponding quantity of secondary incubated bacteria is discharged through valve 21 as product 29.

Further bacterial cultures of solid or liquid form can be fed directly into the secondary phase tank 12 without prior incubation. The rate of addition can be carefully controlled in order to maintain optimal growth in the secondary phase tank 12. A suitable level of nutrient 17 is maintained in the secondary phase tank 12. The secondary phase tank 12 may become a continuous mixing tank where both input (feed) and output (product) streams to the process are carefully controlled in order to maintain the ideal growth equilibrium for a particular bacteria, bacteria strain, or consortium of bacteria. In this manner, the feed and product streams can be continuous or periodic depending on the intended application.

## Claims

1. A system for producing an aqueous bacteria-containing slurry, the system comprising a water supply, at least one temperature control system, and at least two tanks, where at least a first tank is a primary phase tank comprising a heating means, and a second is a secondary phase tank, the primary phase tank being in fluid communication with the secondary phase tank, where the secondary phase tank further comprises a product outlet and a heating means, **characterised in that** the system further comprises a recirculation loop to the secondary phase tank.

2. A system according to Claim 1, where the heating means is connected to the temperature control system.

3. A system according to any preceding claim, where the heating means comprises a heat exchanger, heating jacket, or a heating element such as a thermocouple device.

4. A system according to any preceding claim, where the temperature control to the primary phase tank is independent of the temperature control system for the secondary phase tank.

5. A system according to any preceding claim, further comprising a heat exchanger arranged to heat the water supply before the water is provided to the primary or secondary phase tanks.

6. A system according to any preceding claim, where the at least one primary phase tank includes an inlet for a bacteria-containing material.

7. A system according to any preceding claim, where the at least one secondary phase tank includes mixing apparatus.

8. A system according to any preceding claim, where the at least one primary phase tank includes mixing apparatus.

9. A system according to any preceding claim, the system further comprising a means of aerating the slurry.

10. A system according to Claim 9, where the means for aerating the slurry is arranged in the recirculation loop.

11. A system according to Claim 9 or Claim 10, where the means for aerating the slurry is an eductor.

12. A system according to any preceding claim, where the water inlet to the secondary phase tank is provided with an air-break.

13. A system according to any preceding claim, where the fluid inlet to any or all of the tanks comprises a pair of nozzles which are tangentially arranged within the tank(s).

14. A method of producing an aqueous slurry comprising bacteria, the method comprising:
supplying a source of bacteria to a primary phase tank;
supplying a liquid growth medium to the primary phase tank;
holding the bacteria in the primary phase tank for a length of time at a controlled temperature;
transferring the bacteria-containing liquid to a secondary phase tank;
monitoring and controlling process parameters in the secondary phase tank to optimise bacterial growth; and
discharging product from the secondary phase tank,
**characterised by** recirculating the bacteria-containing liquid to the secondary phase tank.

15. A method according to Claim 14, further comprising the step of aerating the liquid in the secondary phase tank or during recirculation to the secondary phase tank.

## Patentansprüche

1. System zum Erzeugen eines wässrigen, Bakterien enthaltenen Schlamms, wobei das System eine Wasserzuführung, mindestens ein Temperatursteuerungssystem und mindestens zwei Tanks umfasst, wobei mindestens ein erster Tank ein Primärphasentank ist, welcher ein Heizmittel umfasst, und ein zweiter ein Sekundärphasentank ist, wobei der Primärphasentank in Fluidkommunikation mit dem Sekundärphasentank steht, wobei der Sekundärphasentank ferner einen Produktauslass und ein Heizmittel umfasst, **dadurch gekennzeichnet, dass** das System ferner eine Rückführungsschleife in den Sekundärphasentank umfasst.

2. System nach Anspruch 1, wobei das Heizmittel mit dem Temperatursteuerungssystem verbunden ist.

3. System nach einem der vorhergehenden Ansprüche, wobei das Heizmittel einen Wärmetauscher, einen Heizmantel oder ein Heizelement wie beispielsweise eine Thermoelementvorrichtung umfasst.

4. System nach einem der vorhergehenden Ansprüche, wobei die Temperatursteuerung für den Primärphasentank unabhängig von dem Temperatursteuerungssystem für den Sekundärphasentank ist.

5. System nach einem der vorhergehenden Ansprüche, ferner umfassend einen Wärmetauscher, welcher angeordnet ist, um die Wasserzuführung zu erhitzen, bevor das Wasser an den Primär- oder den Sekundärphasentank bereitgestellt wird.

6. System nach einem der vorhergehenden Ansprüche, wobei der mindestens eine Primärphasentank einen Einlass für Bakterien enthaltendes Material einschließt.

7. System nach einem der vorhergehenden Ansprüche, wobei der mindestens eine Sekundärphasentank ein Mischgerät einschließt.

8. System nach einem der vorhergehenden Ansprüche, wobei der mindestens eine Primärphasentank ein Mischgerät einschließt.

9. System nach einem der vorhergehenden Ansprüche, wobei das System ferner ein Mittel zum Belüften des Schlamms umfasst.

10. System nach Anspruch 9, wobei das Mittel zum Belüften des Schlamms in der Rückführungsschleife angeordnet ist.

11. System nach Anspruch 9 oder Anspruch 10, wobei das Mittel zum Belüften des Schlamms ein Eduktor ist.

12. System nach einem der vorhergehenden Ansprüche, wobei der Wassereinlass in den Sekundärphasentank mit einer Luftunterbrechung ausgerüstet ist.

13. System nach einem der vorhergehenden Ansprüche, wobei der Fluideinlass in einem oder allen Tanks ein Paar Düsen umfasst, welche tangential innerhalb des bzw. der Tanks angeordnet ist.

14. Verfahren zur Herstellung eines wässrigen, Bakterien umfassenden Schlamms, wobei das Verfahren Folgendes umfasst:
Zuführen einer Bakterienquelle an einen Primärphasentank;
Zuführen eines flüssigen Wachstumsmediums an den Primärphasentank;
Halten der Bakterien in dem Primärphasentank über einen Zeitraum bei einer gesteuerten Temperatur;
Transferieren der Bakterien enthaltenden Flüssigkeit an einen Sekundärphasentank;
Überwachen und Steuern von Prozessparametern in dem Sekundärphasentank zum Optimieren des Bakterienwachstums; und
Ablassen von Produkt aus dem Sekundärphasentank,
**gekennzeichnet durch** Rückführen des Bakterien enthaltenen Fluids an den Sekundärphasentank.

15. Verfahren nach Anspruch 14, ferner umfassend den Schritt des Belüftens der Flüssigkeit in dem Sekundärphasentank oder während der Rückführung an den Sekundärphasentank.

## Revendications

1. Système pour produire une boue aqueuse contenant des bactéries, le système comprenant une alimentation en eau, au moins un système de contrôle de température et au moins deux réservoirs, dans lequel au moins un premier réservoir est un réservoir de phase primaire qui comprend un moyen de chauffage et un second est un réservoir de phase secondaire, le réservoir de phase primaire étant en communication fluidique avec le réservoir de phase secondaire, dans lequel le réservoir de phase secondaire comprend en outre une sortie de produit et un moyen de chauffage, **caractérisé en ce que** le système comprend en outre une boucle de recirculation au réservoir de phase secondaire.

2. Système selon la revendication 1, dans lequel le moyen de chauffage est connecté au système de contrôle de température.

3. Système selon l'une quelconque des revendications précédentes, dans lequel le moyen de chauffage comprend un échangeur thermique, une enveloppe chauffante ou un élément chauffant tel qu'un dispositif de thermocouple.

4. Système selon l'une quelconque des revendications précédentes, dans lequel le système de contrôle de température pour le réservoir de phase primaire est indépendant du système de contrôle de température pour le réservoir de phase secondaire.

5. Système selon l'une quelconque des revendications précédentes, comprenant en outre un échangeur thermique qui est agencé pour chauffer l'alimentation en eau avant que l'eau ne soit alimentée au réservoir de phase primaire ou secondaire.

6. Système selon l'une quelconque des revendications précédentes, dans lequel l'au moins un réservoir de phase primaire inclut une entrée pour un matériau contenant des bactéries.

7. Système selon l'une quelconque des revendications précédentes, dans lequel l'au moins un réservoir de phase secondaire inclut un appareil de mélange.

8. Système selon l'une quelconque des revendications précédentes, dans lequel l'au moins un réservoir de phase primaire inclut un appareil de mélange.

9. Système selon l'une quelconque des revendications précédentes, le système comprenant en outre un moyen pour aérer la boue.

10. Système selon la revendication 9, dans lequel le moyen pour aérer la boue est agencé dans la boucle de recirculation.

11. Système selon la revendication 9 ou la revendication 10, dans lequel le moyen pour aérer la boue est un déchargeoir.

12. Système selon l'une quelconque des revendications précédentes, dans lequel l'entrée d'eau sur le réservoir de phase secondaire est munie d'un moyen de coupure anti-refoulement.

13. Système selon l'une quelconque des revendications précédentes, dans lequel l'entrée de fluide de l'un quelconque des réservoirs ou de tous les réservoirs comprend une paire d'éjecteurs qui sont agencés de façon tangentielle à l'intérieur du/des réservoir(s).

14. Procédé de production d'une boue aqueuse qui comprend des bactéries, le procédé comprenant :
l'alimentation d'une source de bactéries à un réservoir de phase primaire ;
l'alimentation d'un milieu de croissance liquide au réservoir de phase primaire ;
le maintien des bactéries dans le réservoir de phase primaire pendant une durée temporelle à une température contrôlée ;
le transfert du liquide contenant des bactéries à un réservoir de phase secondaire ;
la surveillance et le contrôle de paramètres de processus dans le réservoir de phase secondaire afin d'optimiser la croissance bactérienne ; et
la décharge du produit depuis le réservoir de phase secondaire ;
**caractérisé par** la recirculation du liquide contenant des bactéries au réservoir de phase secondaire ;

15. Procédé selon la revendication 14, comprenant en outre l'étape d'aération du liquide dans le réservoir de phase secondaire ou pendant la recirculation sur le réservoir de phase secondaire.
